# EUROPÄISCHE PATENTSCHRIFT

(11) **EP 2 324 810 B1**
(45) Veröffentlichungstag und Bekanntmachung des Hinweises auf die Patenterteilung: **29.10.2014**
(21) Anmeldenummer: 09014467.6
(22) Anmeldetag: 19.11.2009
(51) Int. Cl.: A61K 6/083

(54) **Gefüllter Dentalwerkstoff auf der Basis von polymerisationsfähigen Dihydroxyphenylalanin-Derivaten**
Filled dental material based on polymerisable dihydroxyphenylalanine derivatives
Matériau dentaire plein à base de dérivés de dihydroxyphénylalanine résistants à la polymérisation

(43) Veröffentlichungstag der Anmeldung: 25.05.2011
(73) Patentinhaber: Ivoclar Vivadent AG, 9494 Schaan (LI)
(72) Erfinder: Moszner, Norbert, 9492 Triesen (LI); Angermann, Jörg, 7320 Sargans (CH); Rheinberger, Volker, 9490 Vaduz (LI); Fischer, Urs Karl, 9320 Arbon (CH)
(74) Vertreter: UEXKÜLL & STOLBERG

(56) Entgegenhaltungen:
- WO-A1-2006/045034
- WO-A2-2005/118831

## Beschreibung

Die vorliegende Erfindung betrifft Dentalmaterialien auf der Basis von polymerisationsfähigen Dihydroxyphenylalanin-Derivaten, die sich insbesondere als Zemente, Komposite und Beschichtungen eignen.

Die DE 196 43 007 A1 offenbart Haftvermittler und Klebstoffe auf der Basis von klebenden Proteinen, die an polymerisierbare Kunststoffmonomere gekoppelt sind. Ein bevorzugtes klebendes Protein ist das aus den Byssus-Fäden der Miesmuschel isolierte Mytilus edulis foot protein. Die Haftvermittler und Klebstoffe sollen sich für dentale Anwendungen eignen.

Die WO 2006/045034 offenbart selbstätzende dentale Primer, die Dihydroxyphenylalanin (DOPA) in Kombination mit verdünnter Mineralsäure und ggf. olefinisch ungesättigten Monomeren, Aldehyden und Initiatoren für die Lichthärtung enthalten.

Die US 2006/0009550 offenbart die Herstellung von Hydrogelen durch Copolymerisation wässeriger Mischungen von Poly(ethylenglycol)diacrylat und der radikalisch polymerisierbaren DOPA-Derivate N-Methacryloyl-3,4-dihydroxy-L-phenylalanin und N-(13-(N'-t-Boc-L-3',4'-dihydroxyphenylalaninamido)-4,7-10-trioxatridecanyl)-methacrylamid. Die Hydrogele sollen sich als chirurgische Adhäsive für die medizinische oder dentale Anwendung und als Träger für die Wirkstoffabgabe an Schleimhäute eignen. Es wurde gefunden, dass DOPA-Derivate einen inhibierenden Effekt auf die radikalische Polymerisation ausüben.

Die Synthese von N-Methacryloyl-3,4-dihydroxy-L-phenylalanin und N-(13-(N'-t-Boc-L-3',4'-dihydroxyphenylalaninamido)-4,7-10-trioxatridecanyl)-methacrylamid wird in Lee et al., J. Biomater. Sci. Polymer Edn. 15 (2004) 449-464 beschrieben.

L-DOPA ist der unmittelbare biologische Vorläufer von Dopamin (3,4-Dihydroxyphenylethylamin), einem Arzneimittel zur therapeutischen Behandlung von Parkinson. Aus der DE 100 82 749 T1 ist bekannt, dass L-DOPA enthaltende Copolymere von (Meth)acrylsäure den Wirkstoff L-DOPA kontrolliert freisetzen. Dies ist zwangsläufig mit einem Abbau der Polymeren verbunden.

Dentalwerkstoffe auf der Basis von Dihydroxyphenylalanin-Derivaten, die sich als Zemente, Komposite und Beschichtungen eignen, sind aus dem Stand der Technik nicht bekannt.

Der Erfindung liegt die Aufgabe zugrunde, Dentalwerkstoffe bereitzustellen, die sich als Zemente, Komposite und Beschichtungsmaterialien eignen, die sich durch eine gute Haftung an Dentin und Zahnschmelz auszeichnen, die Füllstoffpartikel fest einbinden und die sich durch eine gute Polymerisationsfähigkeit auszeichnen.

Die Aufgabe wird erfindungsgemäss durch Dentalwerkstoffe gelöst, die
a) mindestens eine radikalisch polymerisierbare Verbindung gemäß der allgemeinen Formeln I 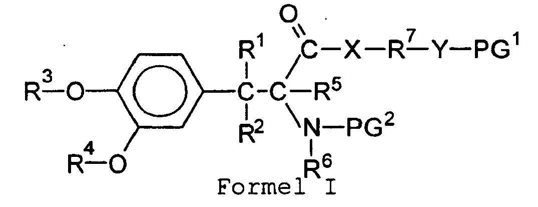 in der
   - R¹, R²: unabhängig voneinander jeweils H oder ein C₁-C₈- Alkyl-Rest sind,
   - R³, R⁴, R⁵: unabhängig voneinander jeweils H oder ein C₁-C₄- Alkyl- Rest sind,
   - R⁶: H, ein linearer oder verzweigter C₁-C₁₀-Alkylrest oder ein C₁-C₁₀-Alkanoylrest ist,
   - R⁷: ein linearer oder verzweigter C₁-C₁₅-Alkylenrest ist oder entfällt, wobei die Kette der Kohlen- stoffatome des Alkylrests durch O- oder S-Atome unterbrochen sein kann, vorzugsweise entfällt oder ein linearer oder verzweigter C₁-C₁₅- Alkylenrest,
   - X, Y: unabhängig voneinander jeweils O oder NR⁸ sind oder entfallen, wobei R⁷ nur dann entfallen kann, wenn auch X und/oder Y entfällt, und wobei R⁷, X und Y nur dann gleichzeitig entfal- len können, wenn PG¹ = H ist,
   - R⁸: H oder ein C₁-C₁₀-Alkyl-Rest ist,
   - PG¹ PG²: unabhängig voneinander jeweils H oder eine radi- kalisch polymerisationsfähige Gruppe, vorzugs- weise eine Vinyl-, Allyl-, (Meth)acryl-Gruppe sind, wobei die beiden Reste PG¹ und PG² nicht gleichzeitig H sein können;
b) Initiator für die radikalische Polymerisation; und
c) Füllstoff enthalten;

Die Formel erfasst nur solche Verbindungen, die mit der chemischen Valenzlehre vereinbar sind.

Der Hinweis, daß ein Rest durch ein Heteroatom wie O unterbrochen sein kann, ist so zu verstehen, daß die O-Atome in die Kohlenstoffkette des Restes eingeschoben werden, d.h. beidseitig von Kohlenstoffatomen begrenzt werden. Die Anzahl der Heteroatome ist daher um mindestens 1 kleiner als die Zahl der Kohlenstoffatome, und die Heteroatome können nicht endständig sein.

Bevorzugte Bedeutungen der Variablen der Formel I sind:
- R¹, R²: unabhängig voneinander jeweils H oder ein C₁-C₃-Alkyl-Rest, vorzugsweise H,
- R³, R⁴: unabhängig voneinander jeweils H oder Methyl, vorzugsweise H,
- R⁵: H oder ein C₁-C₃-Alkyl-Rest, vorzugsweise H,
- R⁶: H, ein linearer oder verzweigter C₁-C₂-Alkylrest oder ein C₂- Alkanoylrest,
- R⁷: ein linearer C₁-C₄-Alkylenrest oder entfällt, wobei die Kette der Kohlenstoffatome des Alkylrests durch O-Atome unterbrochen sein kann, vorzugsweise ein C₁-₉-Alkylrest,
- X, Y: unabhängig voneinander jeweils O oder NR⁸ oder entfallen,
- R⁸: H oder C₁-C₂-Alkyl-Rest,
- PG¹, PG²: unabhängig voneinander jeweils H oder eine (Meth)acryl-Gruppe darstellen, wobei beide Reste nicht gleichzeitig H sein können.

Besonders bevorzugt sind solche Verbindungen gemäß Formel I, in denen PG2 eine radikalisch polymerisationsfähige Gruppe und PG1 H ist, sowie Verbindungen, in denen PG1 und PG2 jeweils eine radikalisch polymerisierbare Gruppe sind.

Die bevorzugten Bedeutungen der Variablen können unabhängig voneinander gewählt werden. Verbindungen, in denen alle Variablen eine der bevorzugten Definitionen aufweisen, sind besonders bevorzugt.

Die erfindungsgemäßen Werkstoffe enthalten vorzugsweise 0,05 bis 40 Gew.-%, besonders bevorzugt 1 bis 30 Gew.-% und ganz besonders bevorzugt 1 bis 20 Gew.-% mindestens einer Verbindung gemäß Formel I. Wenn nicht anders angegeben beziehen sich alle Prozentangaben hierin auf die Gesamtmasse des Dentalwerkstoffs.

Es wurde überraschend gefunden, dass die erfindungsgemässen Werkstoffe nach der Härtung eine hohe Haftung nicht nur an Kollagen sondern auch an dentalem Hartgewebe wie Zahnschmelz und Dentin und an metallischen Substraten aufweisen.

Besonders überraschend war, dass die Werkstoffe gut polymerisierbar sind und nach der Härtung gute mechanische Eigenschaften aufweisen. Dies war nicht zu erwarten, da aus dem Stand der Technik bekannt war, dass DOPA-Derivate die radikalische Polymerisation inhibieren, was bei füllstoffhaltigen Materialien besondere Probleme erwarten ließ, da hier die Polymerisation durch den Füllstoff zusätzlich erschwert wird. Erstaunlicherweise konnte die inhibierende Wirkung des DOPAs aber durch die Zugabe von Füllstoff ausgeglichen werden. Die erfindungsgemäßen füllstoffhaltigen Werkstoffe haben eine höhere Reaktivität als entsprechende ungefüllte Materialien und weisen eine deutlich höhere Polymerisationsfähigkeit auf.

Aus dem Stand der Technik war außerdem war bekannt, dass DOPAhaltige Polymere DOPA freisetzen können, was einerseits nachteilig für die mechanische Stabilität der Polymere ist und andererseits die unerwünschte Freisetzung pharmazeutisch aktiver Substanzen erwarten ließ. Die erfindungsgemäßen Wirkstoffe waren jedoch auch nach Wasserlagerung stabil und setzen keine unerwünschten Komponenten frei.

Polymerisationsfähige Dihydroxyphenylalanin-Derivate gemäß der allgemeinen Formel I sind aus dem Stand der Technik bekannt und lassen sich durch einfache Syntheseverfahren herstellen. Beispielsweise lassen sich Phenyl-D,L-alanin-Derivate (PG¹, PG² und R⁵, R⁶ = H; X und R⁷ entfallen und Y = O) mit (Meth)acrylsäurechlorid (PG²-Cl) zu einem entsprechenden polymerisationsfähigen Phenylalanin-Derivat umsetzen:

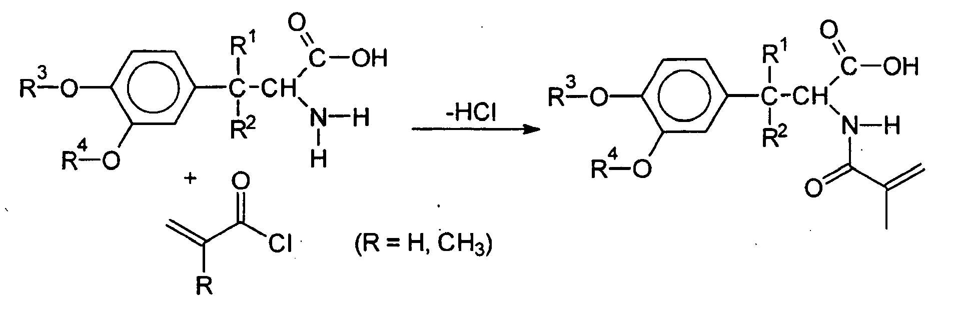

### Konkretes Beispiel:

### Synthese von 3,4-Dihydroxy-N-methacryloyl-DL-phenylalanin:

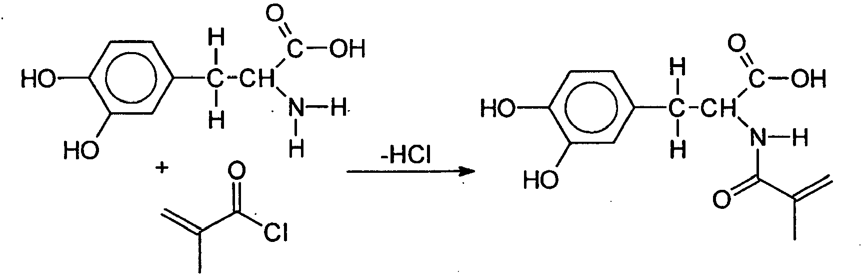

Phenyl-D, L-alanin-Derivate mit den Substituenten R¹, R², R³, R⁴, R⁵ und R⁶ lassen sich mit bekannten Alkylierungs- bzw. Acylierung-Methoden der organischen Chemie und unter Berücksichtigung der Schutzgruppentechnik (vgl. T. W. Greene, Protective Groups in Organic Synthesis, J. Wiley&Sons, New York etc.1980; U. Hacksell, T. Högberg, Protective Groups in Organic Synthesis, Acta Pharm. Suec. 23 (1986) 323-369) herstellen. Mit R¹ bzw. R² substituierte DOPA-Derivate sind z.B. durch die Verwendung von entsprechenden Synthesebausteinen bei der DOPA-Synthese zugänglich. So sind z.B. Alkyl-substituierte DOPA-Derivate (R¹, R² = Alkyl) durch Umsetzung von α-ständig mono- oder dialkylsubstituierten Benzylbromiden mit Glycinestern von Benzophenon-Schiffschen Basen analog zu T. Ooi, M. Kameda, K. Maruoka, J. Amer. Chem. Soc. 125 (2003) 5139 zugänglich. Die Alkylierung der phenolischen OH-Gruppen (R³ und R⁴) kann nach üblichen Methoden erfolgen, beispielsweise durch Alkylierung mit den entsprechenden Alkylchloriden in Gegenwart von KI analog zu T. Kolasa, M. J. Miller, J. Org. Chem. 55 (1990) 4246. Auf diese Weise kann auch eine Alkylierung der Aminogruppe (R⁶) erreicht werden. Die Alkylierung der phenolischen OH-Gruppen (R³ und R⁴) ist auch mit Dialkylsulfaten analog zu A. Suarez, F. Lopez, R. S. Compagnone, Synth. Commun. 23 (1993) 1991 möglich. In α-Stellung alkylierte DOPA-Derivate (R⁵) sind beispielsweise über die Hydrolyse von entsprechend substituierten 2-Imidazolin-5-onen analog der DE 26 58 941 A1 zugänglich.

Bevorzugte Beispiele für die polymerisationsfähigen Dihydroxyphenylalanin-Derivate, der allgemeinen Formel I sind:

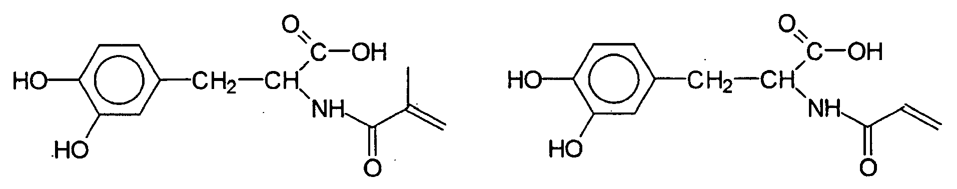

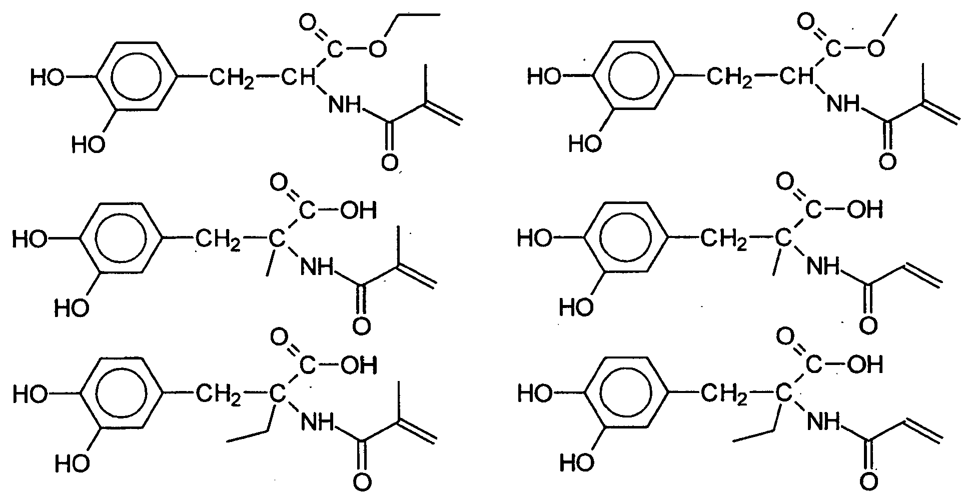

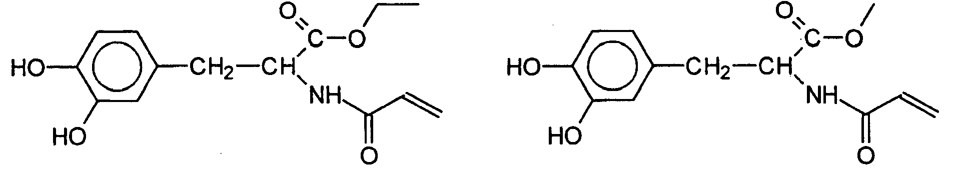

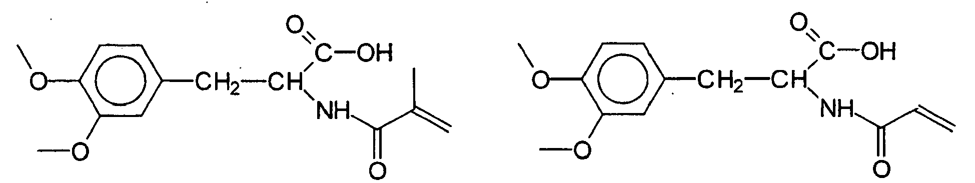

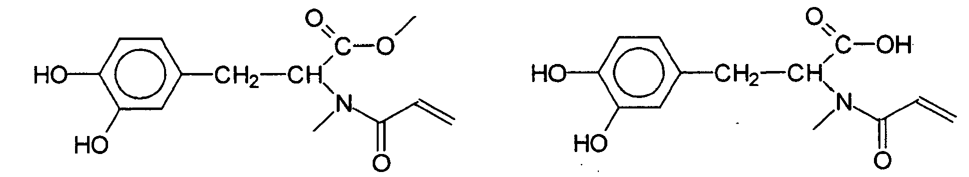

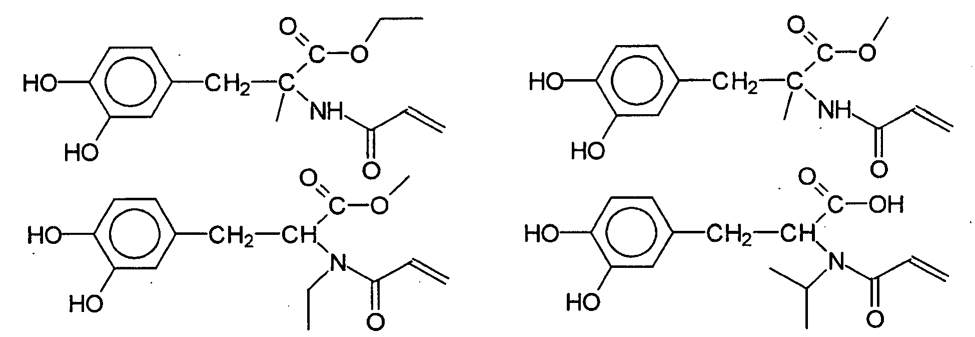

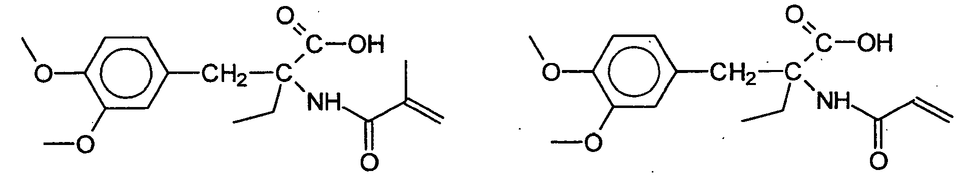

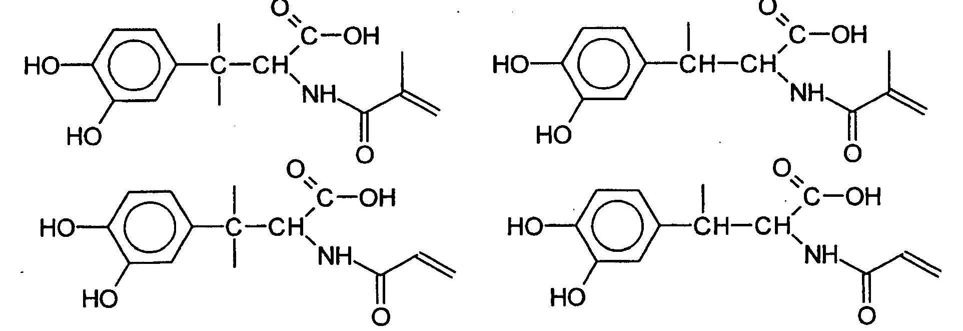

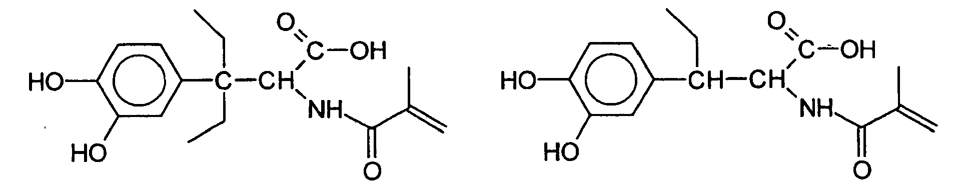

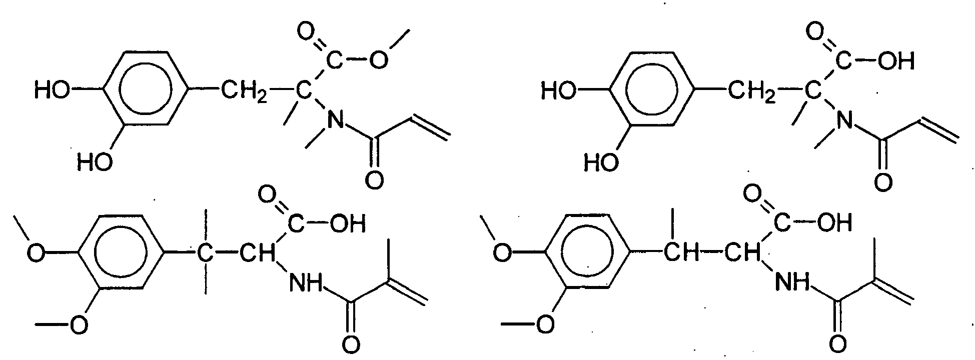

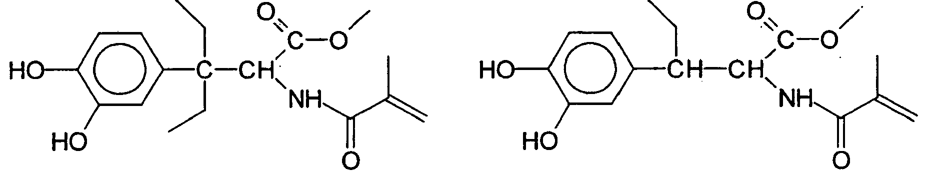

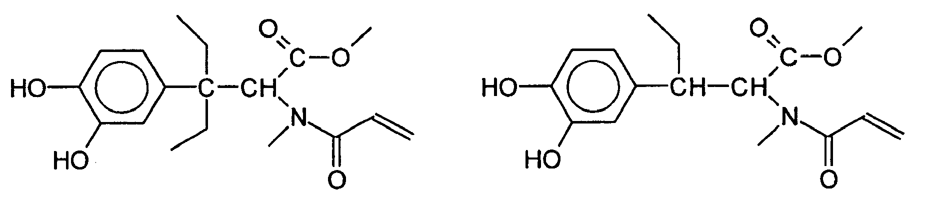

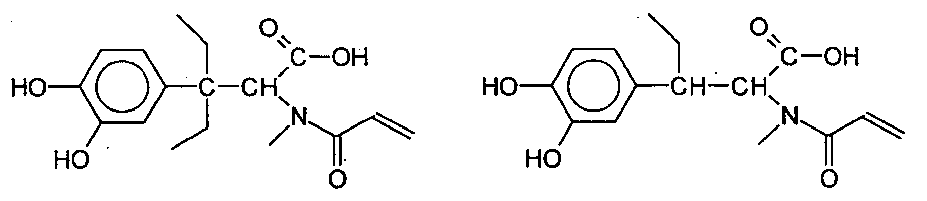

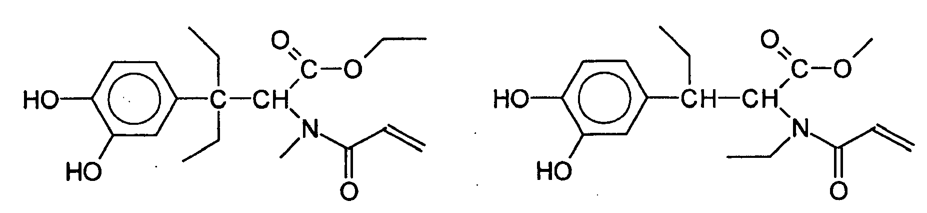

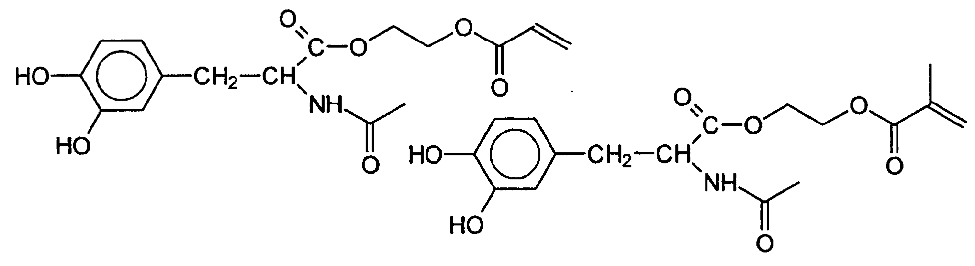

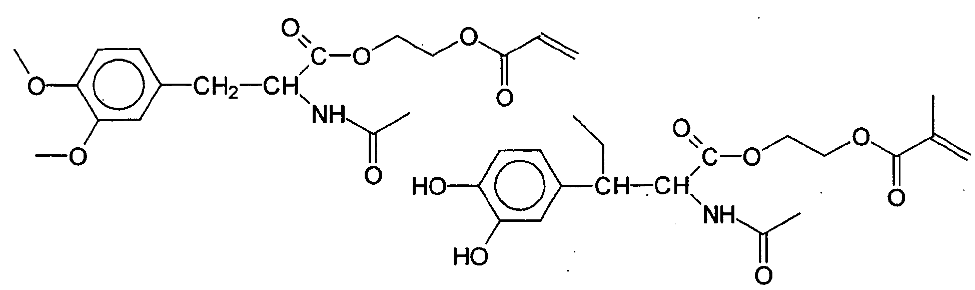

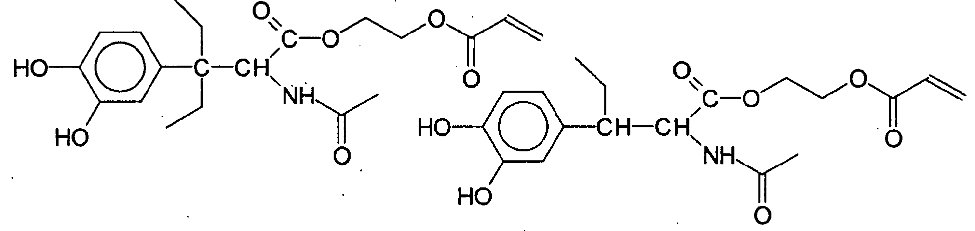

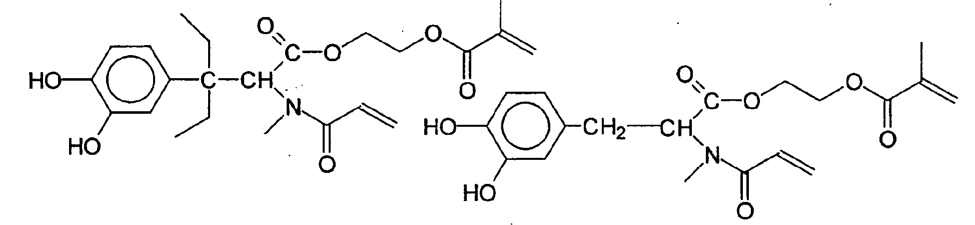

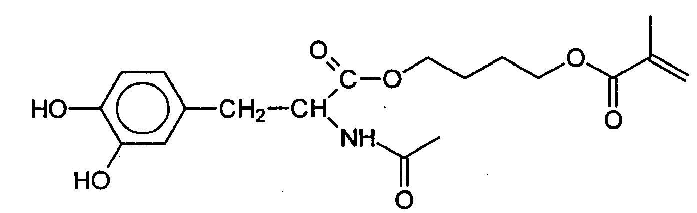

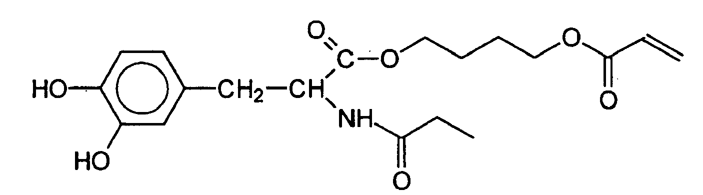

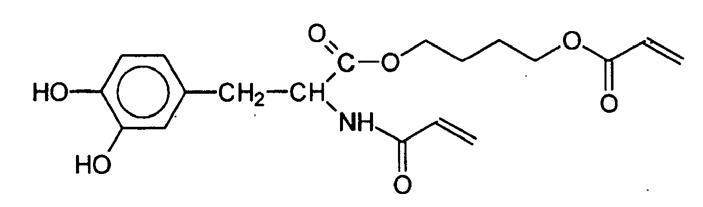

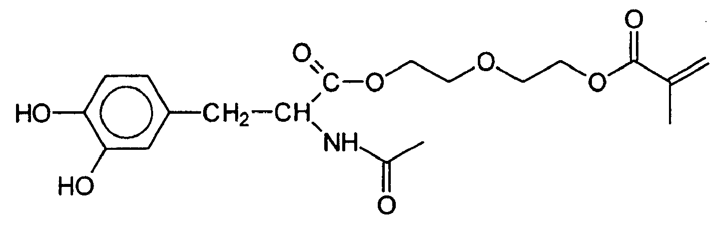

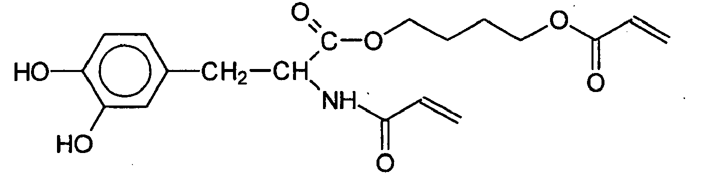

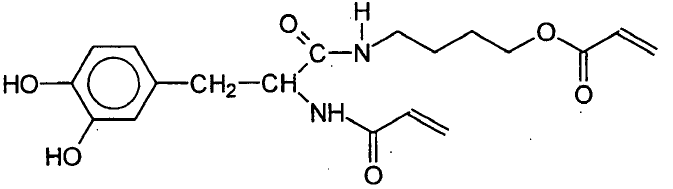

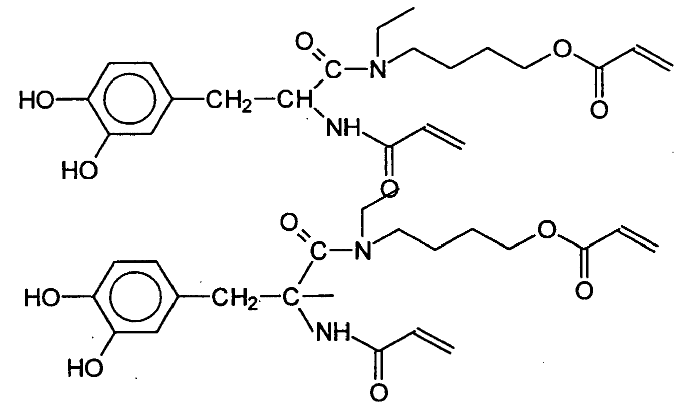

Die polymerisationsfähigen Dihydroxyphenylalanin-Derivate gemäß Formel I sind gut in organischen Lösungsmitteln wie Aceton, Acetonitril oder Ethylacetat löslich und eignen sich somit besonders als Bestandteil von hydrophoben Kompositmatrices. Sie können daher vorteilhaft mit radikalisch polymerisierbaren Monomeren kombiniert werden.

Die erfindungsgemässen Dentalmaterialien enthalten als radikalisch polymerisierbare Monomere vorzugsweise mono- und/oder polyfunktionelle (Meth)acrylate. Unter monofunktionellen Monomeren werden Monomere mit einer, unter polyfunktionellen Monomeren Monomere mit mehr als einer radikalisch polymerisierbaren Gruppe verstanden. Bevorzugte Beispiele sind Methyl-, Ethyl-, Hydroxyethyl-, Butyl-, Benzyl-, Tetrahydrofurfuryl- oder Isobornyl(meth)acrylat, Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)-acrylat, sowie Glycerindi(meth)acrylat, 1,4-Butandioldi(meth)-acrylat, 1,10-Decandioldi(meth)acrylat und 1,12-Dodecandioldi-(meth)acrylat.

Als zusätzliche Monomere lassen sich bevorzugt auch hydrolysestabile Verdünner- und/oder Vernetzermonomere einsetzen. Bevorzugte Verdünnermonomere sind hydrolysestabile Mono(meth)acrylate, z.B. Mesitylmethacrylat, oder 2-(Alkoxymethyl)acrylsäuren, z.B. 2-(Ethoxymethyl)acrylsäure, 2-(Hydroxymethyl)acrylsäure, N-mono- oder N-disubstitiuierte Acrylamide, wie z.B. N-Ethylacrylamid, N,N-Dimethacrylamid, N-(2-Hydroxyethyl)acrylamid, N-Methyl-N-(2-hydroxyethyl)acrylamid, oder N-monosubstituierte Methacrylamide, wie z.B. N-Ethylmethacrylamid oder N-(2-Hydroxyethyl)methacrylamid sowie ausserdem N-Vinylpyrrolidon und Allylether.

Beispiele für bevorzugte hydrolysestabile Vernetzermonomere sind Urethane aus 2-(Hydroxymethyl)acrylsäureestern und Diisocyanaten, wie 2,2,4-Trimethylhexamethylendiisocyanat oder Isophorondiisocyanat, vernetzende Pyrrolidone, wie z.B. 1,6-Bis(3-vinyl-2-pyrrolidonyl)-hexan, oder kommerziell zugängliche Bisacrylamide, wie Methylen- oder Ethylenbisacrylamid, oder Bis(meth)acrylamide, wie z.B. N,N'-Diethyl-1,3-bis(acryl-amido)-propan, 1,3-Bis(methacrylamido)-propan, 1,4-Bis(acryl-amido)-butan oder 1,4-Bis(acryloyl)-piperazin, die durch Umsetzung aus den entsprechenden Diaminen mit (Meth)acrylsäurechlorid synthetisiert werden können.

Weiterhin lassen sich als zusätzliche Monomere auch bekannte schrumpfungsarme radikalisch ringöffnend polymerisierbare Monomere, wie z.B. mono- oder multifunktionelle Vinylcyclopropane und bicyclische Cyclopropanderivate, vorzugsweise die in DE 196 16 183 C2 und EP 03 022 855 beschriebenen, oder cyclische Allylsulfide einsetzen, vorzugsweise die in US 6,043,361 oder US 6,344,556 beschriebenen. Diese Monomere können vorteilhaft auch in Kombination mit den voranstehend aufgeführten Di(meth)acrylat-Vernetzern verwendet werden.

Bevorzugte ringöffnend polymerisierbare Monomere sind Vinylcyclopropane, insbesondere 1,1-Di(ethoxycarbonyl)- oder 1,1-Di(methoxycarbonyl)-2-vinylcyclopropan oder die Ester der 1-Ethoxycarbonyl- oder 1-Methoxycarbonyl-2-vinylcyclopropancarbonsäure mit Ethylenglycol, 1,1,1-Trimethylolpropan, 1,4-Cyclohexandiol oder Resorcin. Bevorzugte bicyclische Cyclopropanderivate sind 2-(Bicyclo[3.1.0]hex-1-yl)acrylsäuremethyl- oder -ethylester oder deren Disubstitutionsprodukte in 3-Stellung, wie (3,3-Bis(ethoxycarbonyl)bicyclo[3.1.0]hex-1-yl)acrylsäuremethyl- oder -ethylester. Bevorzugte cyclische Allylsulfide sind die Additionprodukte von 2-(Hydroxymethyl)-6-methylen-1,4-dithiepan oder 7-Hydroxy-3-methylen-1,5-dithiacyclooctan mit 2,2,4-Trimethylhexamethylen-1,6-diisocyanat oder dem asymmetrischen Trimeren von Hexamethylendiisocyanat (Desmodur^{®} XP2410 der Bayer AG).

Ausserdem können als zusätzliche Monomere radikalisch polymerisierbare Polysiloxane eingesetzt werden, die aus geeigneten Methacrylsilanen, wie z.B. 3-(Methacryloyloxy)propyltrimethoxysilan, hergestellt werden können. Bevorzugt sind die in der DE 199 03 177 C2 beschrieben Polysiloxane.

Schliesslich lassen sich als zusätzliche Monomere auch Mischungen der voranstehend genannten Monomeren mit radikalisch polymerisierbaren, säuregruppenhaltigen Monomeren, sogenannten Haftmonomeren, verwenden. Bevorzugte säuregruppenhaltige Monomere sind polymerisationsfähige Carbonsäuren, wie Maleinsäure, Acrylsäure, Methacrylsäure, 2-(Hydroxymethyl)acrylsäure, 4-(Meth)acryloyloxyethyltrimellitsäureanhydrid, 10-Methacryloyloxydecylmalonsäure, N-(2-Hydroxy-3-methacryloyloxypropyl)-N-phenylglycin und 4-Vinylbenzoesäure. Bevorzugt sind weiterhin Phosphonsäuremonomere wie Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentyl-phosphonsäure, 2-[4-(Dihydroxyphosphoryl)-2-oxa-butyl]-acrylsäure oder 2-[4-(Dihydroxyphosphoryl)-2-oxa-butyl]-acrylsäureethyl- oder - 2,4,6-trimethylphenylester. Bevorzugt sind ausserdem acide polymerisationsfähige Phosphorsäureester wie 2-Methacryloyloxypropylmono- oder -dihydrogenphosphat, 2-Methacryloyloxyethylmono- oder -dihydrogenphosphat, 2-Methacryloyloxyethylphenyl-hydrogenphosphat, Dipentaerythritolpentamethacryloyloxyphosphat, 10-Methacryloyloxydecyl-dihydrogenphosphat, Phosphorsäuremono-(1-acryloyl-piperidin-4-yl)-ester, 6-(Methacrylamido)hexyldihydrogenphosphat und 1,3-Bis-(N-acryloyl-N-propyl-amino)-propan-2-yl-dihydrogenphosphat. Weiter bevorzugte säuregruppenhaltige Monomere sind polymerisationsfähige Sulfonsäuren, wie Vinylsulfonsäure, 4-Vinylphenylsulfonsäure oder 3-(Methacrylamido)propylsulfonsäure.

Die erfindungsgemäßen Dentalwerkstoffe enthalten vorzugsweise keine hydrophilen Monomere, wie beispielsweise Poly(ethylenglycol)di(meth)acrylate, die zu einer Erhöhung der Wasseraufname der Materialien beitragen und damit deren Verwendbarkeit für dentale Zwecke beeinträchtigen.

Besonders bevorzugt sind Dentalwerkstoffe, die als zusätzliches radikalisch polymerisierbares Monomer mindestens ein mono- und/oder polyfunktionelles (Meth)acrylat, Bis-GMA, UDMA, Trimethylolpropantrimethacrylat, Glycerindimethacrylat, 1,10-Decandioldimethacrylat, N,N'-Diethyl-1,3-bis(acrylamido)-propan oder eine Mischung davon enthalten.

Dentalwerkstoffe, die als Komponente (d) mindestens ein weiteres polymerisierbares Monomer enthalten, sind bevorzugt. Besonders bevorzugt sind Werkstoffe, die 5 bis 90 Gew.-% ganz besonders bevorzugt 20 bis 80 Gew.-% ein und/oder mehrere zusätzliche radikalisch polymerisierbare mono- und/oder polyfunktionelle Verdünnner- bzw. Vernetzermonomere enthalten. Weiter bevorzugt sind Werkstoffe, die ausserdem 0 bis 50 Gew.-% ganz besonders bevorzugt 0 bis 30 Gew.-% ein und/oder mehrere radikalisch polymerisierbare, säuregruppenhaltige Monomere enthalten. Besonders bevorzugt sind Werkstoffe, die 5 bis 80 Ges.-% und ganz besonders bevorzugt 20 bis 70 Gew.-% eines oder mehrerer polyfunktioneller Monomere enthalten. Diese Mengenangaben beziehen sich auf die Masse der Monomere in den Werkstoffen.

Der Dentalwerkstoff kann je nach Art des verwendeten Initiators heiß, kalt oder durch Licht polymerisierbar sein. Zur Initiierung der radikalischen Photopolymerisation werden Benzophenon, Benzoin sowie deren Derivate oder α-Diketone oder deren Derivate, wie 9,10-Phenanthrenchinon, 1-Phenyl-propan-1,2-dion, Diacetyl oder 4,4'-Dichlorbenzil, eingesetzt. Bevorzugt werden Campherchinon und 2,2-Dimethoxy-2-phenyl-acetophenon und besonders bevorzugt α-Diketone in Kombination mit Aminen als Reduktionsmittel, wie z.B. 4-(Dimethylamino)-benzoesäureester, N,N-Dimethylaminoethylmethacrylat, N,N-Dimethyl-sym.-xylidin oder Triethanolamin, verwendet. Besonders geeignet sind auch Norrish-Typ-I-Photoinitiatoren, vor allem Acyl- oder Bisacylphosphinoxide, Monoacyltrialkyl- bzw. Diacyldialkylgermanium-Verbindungen, wie z.B. Benzoyltrimethylgermanium, Dibenzoyldiethylgermanium oder Bis(4-methoxybenzoyl)diethylgermanium. Dabei lassen sich auch Mischungen verschiedener Photoinitiatoren einsetzen, wie z.B. Dibenzoyldiethylgermanium in Kombination mit Campherchinon und 4-Dimethylaminobenzoesäureethylester.

Als Initiatoren für die Heisshärtung eignen sich besonders Benzpinakol und 2,2'-Dialkylbenzpinakole. Als Initiatoren für eine bei Raumtemperatur durchgeführte Polymerisation werden Redox-Initiatorkombinationen, wie z.B. Kombinationen von Benzoylperoxid mit N,N-Dimethyl-sym.-xylidin oder N,N-Dimethyl-p-toluidin, verwendet. Darüber hinaus sind auch Redoxsysteme bestehend aus Peroxiden und solchen Reduktionsmitteln, wie z.B. Ascorbinsäure, Barbituraten oder Sulfinsäuren, besonders geeignet.

Erfindungsgemäß sind solche Werkstoffe bevorzugt, die einen Photoinitiator, insbesondere einen Photoinitiator auf Basis von Campherchinon/Amin, einem Bisacylphosphinoxid, einer Diacyldialkylgermanium-Verbindung oder eine Mischung davon enthalten.

Die erfindungsgemäßen Dentalwerkstoffe enthalten vorzugsweise 0,01 bis 10 Gew.-%, bevorzugt 0,1 bis 3,0 Gew.-% an Initiator und ggf. Aktivator.

Weiterhin enthalten die erfindungsgemäss eingesetzten Zusammensetzungen zur Verbesserung der mechanischen Eigenschaften oder zur Einstellung der Viskosität organische oder anorganische Füllstoffpartikel. Die Füllstoffpartikel weisen vorzugsweise eine mittlere Teilchengröße [bestimmt durch Transmissions- (10-80 nm) oder Rasterelektronenmikroskopie (50 nm bis 5 µm) bzw. Laserbeugung (0,1 bis 100 µm)] von 10 nm bis 50 µm, besonders bevorzugt 10 nm bis 30 µm und ganz besonders bevorzugt 10 nm bis 5 µm auf.

Bevorzugte anorganische partikuläre Füllstoffe sind amorphe kugelförmige Materialien auf der Basis von Oxiden, wie ZrO₂ und TiO₂ bzw. Mischoxiden aus SiO₂, ZrO₂ und/oder TiO₂ mit einer mittleren Partikelgröße von 10 nm bis 1 µm, nanopartikuläre oder mikrofeine Füllstoffe, wie pyrogene Kieselsäure oder Fällungskieselsäure mit einer mittleren Partikelgröße von 10 nm bis 500 nm sowie Minifüllstoffe, wie Quarz-, Glaskeramik- oder Glaspulver mit einer mittleren Partikelgrösse von 0,1 bis 5 µm, vorzugsweise 0,2 bis 3 µm und ganz besonders bevorzugt 0,4 bis 1,5 µm, sowie röntgenopake Füllstoffe, wie Ytterbiumtrifluorid oder nanopartikuläres Tantal(V)-oxid bzw. Bariumsulfat mit einer mittleren Partikelgrösse von 10 nm bis 500 nm.

Die erfindungsgemäßen Dentalmaterialien enthalten vorzugsweise 1 bis 90 Gew.-%, bevorzugt 1 bis 85 Gew.-% und ganz bevorzugt 1 bis 20 Gew.-% oder 20 bis 85 Gew.-% Füllstoff, wobei der Füllstoffgehalt vom gewünschten Verwendungszweck der Werkstoffe abhängt. Dabei tragen die Füllstoffe wesentlich zur Aushärtung der Materialien bei.

Gegebenenfalls können die erfindungsgemäss eingesetzten Zusammensetzungen weitere Additive enthalten, vor allem Lösungsmittel, wie Aceton, Ethylacetat und Mischungen davon, sowie Stabilisatoren, Aromastoffe, Farbmittel, mikrobiocide Wirkstoffe, fluoridionenabgebende Additive, optische Aufheller, Weichmacher oder UV-Absorber. Die erfindungsgemäßen Dentalwerkstoffe enthalten vorzugsweise kein Wasser, d.h. maximal 0,5 Gew.-% Wasser.

Gemäß einer weiter bevorzugten Ausführungsform enthalten die erfindungsgemässen Werkstoffe Borsäure, vorzugsweise in einer Menge von 0 bis 2,0 Moläquivalent bezogen auf die Verbindung gemäss Formel I, besonders bevorzugt 0 bis 1,0 Moläquivalent. Es wurde gefunden, dass Borsäure, insbesondere im Falle von R³ = R⁴ = H, die inhibierende Wirkung von DOPA-Derivaten auf die radikalische Polymerisation verringert. Daher kann bei der Verwendung von Borsäure auf die Zugabe von Füllstoff verzichtet werden, wobei Werkstoffe, die Füllstoff und Borsäure enthalten, bevorzugt sind.

Zusammensetzungen, die als Komponente (e) 0 bis 95 Gew.-%, besonders bevorzugt 0 bis 70 Gew.-% und ganz besonders bevorzugt 5 bis 50 Gew.-% nicht-wässeriges Lösungsmittel enthalten sind erfindungsgemäß besonders geeignet.

Die erfindungsgemäßen Dentalwerkstoffe enthalten vorzugsweise:
a) 0,05 bis 40 Gew.-%, bevorzugt 1 bis 30 Gew.-% und besonders bevorzugt 1 bis 20 Gew.-% mindestens einer Verbindung gemäß Formel I;
b) 0,01 bis 10 Gew.-%, bevorzugt 0,1 bis 3,0 Gew.-% an Initiator;
c) 1 bis 85 Gew.-%, bevorzugt 1 bis 20 Gew.-% (zur Verwendung als Beschichtungsmaterial) oder 20 bis 85 Gew.-% (zur Verwendung als Zement oder Komposit) an Füllstoff;
d) 0 bis 90 Gew.-%, bevorzugt 0 bis 80 Gew.-% und besonders bevorzugt 5 bis 80 Ges.-% mindestens eines zusätzlichen Monomers;
e) 0 bis 95 Gew.-%, bevorzugt 0 bis 70 Gew.-% und besonders bevorzugt 5 bis 5.0 Gew.-% Lösungsmittel.

Die erfindungsgemäßen Dentalwerkstoffe eignen sich besonders als Zemente, Kompositmaterialien, Füllmaterialien, Adhäsive und als Beschichtungsmaterialien.

Die Erfindung wird im Folgenden anhand von Beispielen näher erläutert.

### Ausführungsbeispiele

### Beispiel 1:

### Synthese von polymerisationsfähigen 3,4-Dihydroxy-DL-phenylalaninen (DL-DOPA)

### Allgemeine Vorschrift zur N-Acylierung von DL-DOPA:

Ein Gemisch aus 19,72 g (0,10 mol) *DL*-DOPA, 64,56 g (0, 40 mol) Hexamethyldisilazan und 4,00 ml Trimethylchlorsilan wurde für 3 h bei 120 °C Badtemperatur gerührt. Flüchtige Bestandteile wurden anschliessend bei 70 °C/20 mbar entfernt. Der Rückstand wurde mit 50 ml Xylol versetzt und erneut eingeengt (70 °C/20 mbar). Die so erhaltene Zwischenstufe wurde in 200 ml Methylenchlorid gelöst, bei -70 °C mit einer Lösung von 0,10 mol Säurechlorid in 20 ml Methylenchlorid versetzt und unter langsamer Erwärmung auf Raumtemperatur 2 d gerührt. Nach dem Einengen im Vakuum (40 °C/600 mbar) wurde der Rückstand mit 500 ml *tert*-Butanol und 100 ml Wasser versetzt, für 30 min bei Raumtemperatur gerührt, mit Natriumsulfat getrocknet, filtriert und das Lösungsmittel abdestilliert (40 °C/20 mbar). Zur Reinigung wurde das_Rohprodukt in 250 ml Ethylacetat 1 h unter Rückfluß erhitzt und von ungelöstem Material abfiltriert. Das klare, gelbe Filtrat wurde vom Lösungsmittel befreit (40 °C/180 mbar) und anschliessend bei 0.1 mbar bis zur Gewichtskonstanz getrocknet.

### a) 3,4-Dihydroxy-N-methacryloyl-DL-phenylalanin (MADOPA)

Unter Verwendung von Methacrylsäurechlorid wurde MADOPA erhalten. Ausbeute: 14.8 g (56 %), gelblicher Feststoff (Schmp.: 63-65 °C). Reinheit (HPLC): 96.16 %

¹H-NMR (400 MHz, DMSO-d₆) δ = 1.79 (s, 3H, CH₃), 2.76-2.92 (m, 2H, CH₂), 4.30-4.36 (m, 1H, NCH), 5.31, 5.62 (2 s, je 1H, =CH₂), 6.47, 6.58, 6.60-6.61 (dd, *J* = 8 Hz, 2 Hz, 1H; s, 1H; m, 1H, =CH'), 7.93 (d, *J* = 8 Hz, 1H, NH), 8.71 (br. s, 2H, OH), 12.5 (br. s, 1H, COOH).

¹³C-NMR (100 MHz, DMSO-d₆) : δ = 18.5 (CH₃) , 35.7 (CH₂) , 54. 1 (NCH), 115.2, 116. 4, 119.8 *(=CH_{Aromat}*), 119.5 (C=CH₂) , 128.7, (=C*_{Aromat}*) , 139.4 (C=CH₂), 143.7, 144.8 (HO-C=) , 167.5 (CONH), 173.2 (COOH).

IR (Diamant-ATR): ∨ = 3202 (br, m, N-H, O-H), 2933 (m, CH₂, CH₃), 1717 (s, C=O*_{Säure}*) , 1652 (s, C=O*_{Amid}*), 1604 (s, C=C), 1532 (s, N-H), 1444 (s, Aromat), 1422 (s, CH₂, CH₃) , 1372 (m, CH₃) , 1194 (s, C-OH), 1116 (s, C-N), 873 cm⁻¹ (s, =CH-) .

### b) N-Acryloyl-3,4-dihydroxy-DL-phenylalanin (ADOPA)

Unter Verwendung von Acrylsäurechlorid wurde ADOPA erhalten. Ausbeute: 14.8 g (56 %), gelblicher Feststoff (Schmp.: 143-145 °C). Reinheit (HPLC): 95.84 %.

¹H-NMR (400 MHz, DMSO-d₆) : δ = 2.69-2.92 (m, 2H, CH₂), 4.39-4.44 (m, 1H, NCH), 5.57-5.60, 6.04-6.08, 6.26-6.32 (3 m, je 1H, HC=CH₂), 6.47, 6.61 (d, *J* = 8 Hz, 1H; s, 2H, =CH*_{Aromat}*) , 8.35 (d, *J* = 8 Hz, 1H, NH), 8.70, 8.74 (2 br. s, je 1H, OH), 12.7 (br. s, 1H, COOH).

¹³C-NMR (100 MHz, DMSO-d₆) : δ = 36.2 (CH₂), 53.8 (NCH), 115.3, 116.3, 119.7 (=CH*_{Aromat}*), 125.6 (HC=CH₂), 128.2, (=C*_{Aromat}*), 131.3 (HC=CH₂), 143.8, 144.9 (HO-C=), 164.4 (CONH), 173.0 (COOH). IR (Diamant-ATR): v = 3300 (br, m, N-H, O-H), 2920 (m, CH₂), 1717 (s, C=O*_{Säure}*) , 1653 (s, C-O*_{Amid}*), 1601 (s, C=C), 1516 (ss, N-H), 1444 (s, Aromat), 1414 (s, CH₂) , 1191 (s, C-OH), 1114 (s, C-N), 969 (s, =CH), 871 cm⁻¹ (s, =CH).

### Beispiel 2:

### Radikalische Homopolymerisation von MADOPA in Lösung

Das Monomer MADOPA (2.0 mol/l) wurde in DMF mit 2,2'-Azobisisobutyronitril (AIBN, 2.0 mol-%) bei 65 °C polymerisiert. Nach 5 h wurde die Polymerisation abgebrochen und das Polymerisat aus der 10-fachen Menge Diethylether ausgefällt, abfiltriert und bis zur Gewichtskonstanz im Feinvakuum bei 50 °C getrocknet. Es wurde ein weisses Polymer in nahezu quantitativer Ausbeute erhalten. Die Polymerstruktur konnte mittels ¹H-NMR-Spektroskopie bestätigt werden.

NMR-Spektroskopische Daten für Poly(MADOPA):

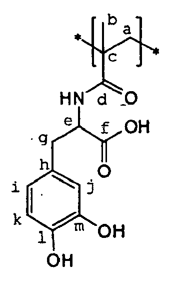

¹H-NMR (400 MHz, MeOD, δ in ppm) : 0.88 (Hₐ, H_{b}, 5H), 2.6-3.1 (H_{g}, 2H), 4.57 (Hₑ, 1H) , 6.75 (Hᵢ, Hⱼ , Hₖ, 3H) , 8.0 (H_{NH}, 1H)

¹³C-NMR (100 MHz, MeOD, δ in ppm) : 19.4 C_{b}, 37.9 C_{g}, 46.8 C_{c}, 48.9 Cₑ, 56.2 Cₐ, 117.7 C_{j/k}, 122.3 Ci, 129.9 Ch, 145.2 Cₗ, 146.2 Cₘ, 175.1 C_{f}, 178.9 C_{d}

### Beispiel 3:

### Radikalische Copolymerisation von ADOPA mit MMA in Lösung

Das Monomer ADOPA (0.2 mol/l) und Methylmethacrylat (MMA) (1,8 mol/l) wurden in DMF mit 2,2'-Azobisisobutyronitril (AIBN, 2.0 mol-%) bei 65 °C polymerisiert. Nach 5 h wurde die Polymerisation abgebrochen und das Polymerisat aus der 10-fachen Menge Methanol ausgefällt, abfiltriert und bis zur Gewichtskonstanz im Feinvakuum getrocknet. Es wurde ein weisses Polymer in nahezu quantitativer Ausbeute erhalten. Die Polymerstruktur konnte mittels ¹H-NMR-Spektroskopie bestätigt werden. Es ergab sich eine Copolymerstruktur mit 18 mol-% ADOPA-Bausteinen.

### Beispiel 4:

### Herstellung von Kompositzementen auf der Basis von MADOPA

Entsprechend der nachfolgend aufgeführten Tabelle 1 wurden Kompositbefestigungszemente auf der Basis einer Dimethacrylat-Mischung mit dem polymerisierbaren DOPA-Derivat MADOPA (Zement A) und dem monofunktionellen Benzylmethacrylat (Zement B, Vergleichsbeispiel) sowie Bis-(4-methoxybenzoyl)diethylgermanium als Photoinitiator mittels eines Walzenstuhles "Exakt" (Exakt Apparatebau, Norderstedt) hergestellt. Von den Materialien wurden Prüfkörper (Stäbchen mit einer Länge von 25 mm und einem quadratischen Querschnitt von 2 mm x 2 mm) präpariert, die 2 mal 3 Minuten mit einer dentalen Lichtquelle (Spectramat^{®}, Ivoclar Vivadent AG) bestrahlt und ausgehärtet wurden. Nach der ISO-Norm ISO-4049 (Dentistry - Polymer-based filling, restorative and luting materials) erfolgte die Bestimmung der Biegefestigkeit und des Biege-E-Moduls. Die Ergebnisse sind in der Tabelle 2 zusammengefasst.

Das Beispiel zeigt, dass gefüllte DOPA-monomerhaltige Rezepturen zu Kompositzementen mit guten mechanischen Eigenschaften führen, während analoge nichtgefüllte Harze keine brauchbaren Materialien ergeben.

**Tabelle 1: Zusammensetzung der Kompositzemente**

| **Komponente** | **Zement A [Gew.-%]** | **Harz A*) [Gew.-%]** | **Zement B*) [Gew.-%]** |
|---|---|---|---|
| Bis-(4-methoxybenzoyl)diethylgermanium | 0.58 | 1.45 | 0.58 |
| UDMA¹⁾ | 27.64 | 69.10 | 27.64 |
| Triethylenglycoldimethacrylat | 7.81 | 19.53 | 7.81 |
| MADOPA (Bsp.1a) | 3.90 | 9.92 | - |
| Benzylmethacrylat | - | - | 3.90 |
| pyrogene Kieselsäure²⁾ | 41.34 | - | 41.34 |
| Ytterbiumtrifluorid (Rhone-Poulenc) | 18.73 | - | 18.73 |

| | | | |
|---|---|---|---|
| ^{*)} Vergleich ¹⁾ Additionsprodukt aus 2 mol 2-Hydroxyethylmethacrylat und 1 mol 2,2,4-Trimethylhexamethylendiisocyanat ²⁾ Aerosil OX-50 (Degussa) | | | |

**Tabelle 2: Mechanische Eigenschaften der Kompositzemente**

| **Eigenschaft** | **Zement A** | **Harz A*)** | **Zement B*)** |
|---|---|---|---|
| Biegefestigkeit (MPa) nach 24 h WL¹⁾ | 114 | 32 | 109 |
| E-Modul (MPa) nach 24 h WL¹⁾ | 5280 | 600 | 5200 |

| | | | |
|---|---|---|---|
| ^{*)} Vergleich ¹⁾ WL = Wasserlagerung der Prüfkörper bei 37°C | | | |

### Beispiel 5:

### Herstellung eines Komposites auf der Basis von MADOPA

Unter Verwendung eines Kneters der Firma Linden wurden dentale Komposite mit der in Tabelle 3 angegebenen Zusammensetzung hergestellt. Die Untersuchung der mechanischen Eigenschaften erfolgte analog zu Beispiel 4 und sind in Tabelle 4 zusammengestellt.

**Tabelle 3: Zusammensetzung der Komposite**

| **Komponente** | **Komposit C [Gew.-%]** | **Komposit D*) [Gew.-%]** |
|---|---|---|
| Bis-(4-methoxybenzoyl)diethylgermanium | 0.22 | 0.22 |
| UDMA¹⁾ | 6.72 | 6.72 |
| Triethylenglycoldimethacrylat | 1.77 | 6.72 |
| MADOPA | 1.81 | - |
| Benzylmethacrylat | - | 1.81 |
| Ytterbiumtrifluorid (Rhone-Poulenc) | 14.89 | 14.89 |
| Sphärosil, silanisiert (Tokoyama Soda) ²⁾ | 14.39 | 14.39 |
| Glasfüller GM27884 (Schott) ³⁾ | 51.61 | 51.61 |
| Aerosil OX-50 (Degussa) | 1.00 | 1.00 |

| | | |
|---|---|---|
| ^{*)} Vergleich ¹⁾ Additionsprodukt aus 2 mol 2-Hydroxyethylmethacrylat und 1 mol 2,2,4-Trimethylhexamethylendiisocyanat ²⁾ SiO₂-ZrO₂-Mischoxid (mittlere Primärpartikelgrösse: 250 nm) ³⁾ Silanisierter Ba-Al-Borosilikatglasfüller mit einer mittleren Partikelgrösse von 1,5 µm | | |

**Tabelle 4: Mechanische Eigenschaften der Komposite**

| **Eigenschaft** | **Komposit C** | **Komposit D*)** |
|---|---|---|
| Biegefestigkeit (MPa) nach 24 h WL¹⁾ | 158 | 195 |
| E-Modul (MPa) nach 24 h WL¹⁾ | 1500 | 16200 |

| | | |
|---|---|---|
| ^{*)} Vergleich ¹⁾ WL = Wasserlagerung der Prüfkörper bei 37°C | | |

## Patentansprüche

1. Polymerisierbarer Dentalwerkstoff der,
a) mindestens eine radikalisch polymerisierbare Verbindung gemäß der allgemeinen Formeln I 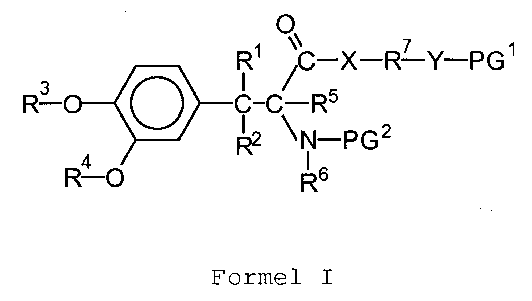 in der
R¹, R² unabhängig voneinander jeweils H oder ein C₁-C₈-Alkyl-Rest sind,
R³, R⁴, R⁵ unabhängig voneinander jeweils H oder ein C₁-C₄-Alkyl- Rest sind,
R⁶ H, ein linearer oder verzweigter C₁-C₁₀-Alkylrest oder ein C₁-C₁₀-Alkanoylrest ist,
R⁷ ein linearer oder verzweigter C₁-C₁₅-Alkylenrest ist oder entfällt, wobei die Kette der Kohlenstoffatome des Alkylrests durch O- oder S-Atome unterbrochen sein kann, vorzugsweise entfällt oder ein linearer oder verzweigter C₁-C₁₅-Alkylenrest,
X, Y unabhängig voneinander jeweils O oder NR⁸ sind oder entfallen,
wobei R⁷ nur dann entfallen kann, wenn auch X und/oder Y entfällt, und
wobei R⁷, X und Y nur dann gleichzeitig entfallen können, wenn PG¹ = H ist,
R⁸ H oder ein C₁-C₁₀-Alkyl-Rest ist,
PG¹, PG² unabhängig voneinander jeweils H oder eine radikalisch polymerisationsfähige Gruppe sind, wobei die beiden Reste PG¹ und PG² nicht gleichzeitig H sein können;
b) Initiator für die radikalische Polymersisation; und
c) Füllstoff enthält.

2. Dentalwerkstoff nach Anspruch 1, in dem PG¹ und PG² unabhängig voneinander jeweils H, eine Vinyl-, Allyl- oder (Meth)acryl-Gruppe sind.

3. Dentalwerkstoff nach Anspruch 1 oder 2, der zusätzlich d) weiteres polymerisierbares Monomer enthält.

4. Dentalwerkstoff nach einem der Ansprüche 1 bis 3, der zusätzlich
e) Lösungsmittel enthält.

5. Dentalwerkstoff nach einem der vorhergehenden Ansprüche, der
a) 0,05 bis 40 Gew.-% mindestens einer Verbindung gemäß Formel I;
b) 0,01 bis 10 Gew.-% an Initiator;
c) 1 bis 85 Gew.-% (Beschichtungsmaterial) oder 20 bis 85 Gew.-% (Zement, Komposit) an Füllstoff;
d) 0 bis 90 Gew.-% mindestens eines zusätzlichen Monomers;
e) 0 bis 95 Gew.-% Lösungsmittel enthält.

6. Dentalwerkstoff nach Anspruch 5, der
a) 1 bis 30 Gew.-% mindestens einer Verbindung gemäß Formel I;
b) 0,1 bis 3,0 Gew.-% an Initiator;
c) 1 bis 20 Gew.-% (Beschichtungsmaterial) oder 20 bis 85 Gew.-% (Zement, Komposit) an Füllstoff;
d) 0 bis 80 Gew.-% mindestens eines zusätzlichen Monomers;
e) 0 bis 70 Gew.-% Lösungsmittel enthält.

7. Dentalwerkstoff nach einem der vorhergehenden Ansprüche, in dem die Variablen der Formel I die folgenden Bedeutungen haben:
R¹, R² unabhängig voneinander jeweils H oder ein C₁-C₃-Alkyl-Rest,
R³, R⁴ unabhängig voneinander jeweils H oder Methyl,
R⁵ H oder ein C₁-C₃-Alkyl-Rest,
R⁶ H, ein linearer oder verzweigter C₁-C₂-Alkylrest oder C₂- Alkanoylrest,
R⁷ ein linearer C₁-C₄-Alkylenrest oder entfällt, wobei die Kette der Kohlenstoffatome des Alkylrests durch O-Atome unterbrochen sein kann,
X, Y unabhängig voneinander jeweils O oder NR⁸ oder entfallen,
R⁸ H oder C₁-C₂-Alkyl-Rest,
PG¹, PG² unabhängig voneinander jeweils H oder eine (Meth)acryl-Gruppe, wobei beide Reste nicht gleichzeitig H sein können.

8. Dentalwerkstoff nach Anspruch 7, in dem die Variablen der Formel I die folgenden Bedeutungen haben:
R¹, R² jeweils H,
R³, R⁴ jeweils H,
R⁵ H,
R⁶ H, ein linearer oder verzweigter C₁-C₂-Alkylrest oder C₂- Alkanoylrest,
R⁷ ein linearer C₁-C₄-Alkylenrest,
X, Y unabhängig voneinander jeweils O oder NR⁸ oder entfallen,
R⁸ H oder C₁-C₂-Alkyl-Rest,
PG¹, PG² unabhängig voneinander jeweils H oder eine (Meth)acryl-Gruppe, wobei beide Reste nicht gleichzeitig H sein können.

9. Dentalwerkstoff nach einem der vorhergehenden Ansprüche, der als zusätzliches radikalisch polymerisierbares Monomer mindestens ein mono- und/oder polyfunktionelles (Meth)acrylat, Bis-GMA, UDMA, Trimethylolpropantrimethacrylat, Glycerindimethacrylat, 1,10-Decandioldimethacrylat, N,N'-Diethyl-1,3-bis(acrylamido)-propan oder eine Mischung davon enthält.

10. Dentalwerkstoff nach einem der vorhergehenden Ansprüche, der frei von hydrophilen Monomeren ist.

11. Dentalwerkstoff nach einem der vorhergehenden Ansprüche, der Füllstoff mit einer mittlere Teilchengröße von 10 nm bis 50 µm enthält.

12. Dentalwerkstoff nach einem der vorhergehenden Ansprüche, der als Füllstoff amorphe kugelförmige Materialien auf der Basis von Oxiden oder Mischoxiden mit einer mittleren Partikelgröße von 10 nm bis 1 µm, nanopartikuläre oder mikrofeine Füllstoffe mit einer mittleren Partikelgröße von 10 nm bis 500 nm, Minifüllstoffe mit einer mittleren Partikelgröße von 0,1 bis 5 µm, oder röntgenopake Füllstoffe mit einer mittleren Partikelgröße von 10 nm bis 500 nm enthält.

13. Dentalwerkstoff nach einem der vorhergehenden Ansprüche, der maximal 0,5 Gew.-% Wasser enthält.

14. Dentalwerkstoff nach einem der vorhergehenden Ansprüche, der Borsäure enthält.

15. Verwendung eines Dentalwerkstoffs gemäß einem der vorhergehenden Ansprüche zur Herstellung eines Zements, Kompositmaterials, Füllmaterials, Adhäsivs oder Beschichtungsmaterials.

## Claims

1. Polymerisable dental material, comprising
a) at least one radically polymerizable compound according to the general formula I 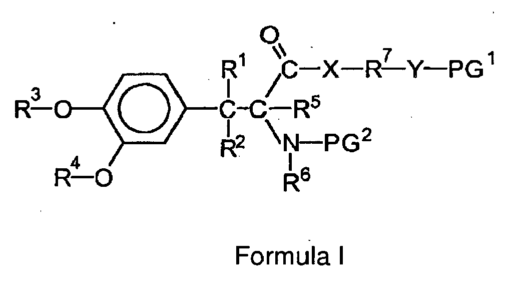 in which
R¹, R² independently of one another are each H or a C₁-C₈ alkyl residue,
R³, R⁴, R⁵ independently of each other are each H or a C₁-C₄ alkyl residue,
R⁶ is H, a linear or branched C₁-C₁₀ alkyl residue or a C₁-C₁₀ alkanoyl residue,
R⁷ is a linear or branched C₁-C₁₅ alkylene residue or is absent, wherein the chain of the carbon atoms of the alkyl residue can be interrupted by O or S atoms, is preferably absent or is a linear or branched C₁-C₁₅ alkylene residue,
X, Y independently of one another are each O or NR⁸ or are absent, wherein R⁷ can only be absent if X and/or Y is also absent, and wherein R⁷, X and Y can be simultaneously absent only if PG¹ = H,
R⁸ is H or a C₁-C₁₀ alkyl residue,
PG¹, PG² independently of one other are each H or a radically polymerisable group, wherein the two residues PG¹ and PG² cannot simultaneously be H;
b) initiator for radical polymerisation; and
c) filler.

2. Dental material according to claim 1, in which PG¹ and PG² independently of one another are each H, a vinyl, allyl or (meth)acrylic group.

3. Dental material according to claim 1 or 2 further comprising
d) an additional polymerisable monomer.

4. Dental material according to one of claims 1 to 3 further comprising
e) solvent.

5. Dental material according to one of the preceding claims comprising
a) 0.05 to 40 wt % of at least one compound according to Formula I;
b) 0.01 to 10 wt % initiator;
c) 1 to 85 wt % (coating material) or 20 to 85 wt % (cement, composite) filler;
d) 0 to 90 wt % of at least one additional monomer;
e) 0 to 95 wt % solvent.

6. Dental material according to claim 5 comprising
a) 1 to 30 wt % of at least one compound according to Formula I;
b) 0.1 to 3.0 wt % initiator;
c) 1 to 20 wt % (coating material) or 20 to 85 wt % (cement, composite) filler;
d) 0 to 80 wt % of at least one additional monomer;
e) 0 to 70 wt % solvent.

7. Dental material according to one of the preceding claims in which the variables of Formula I have the following meanings:
R¹, R² independently of one another are each H or a C₁-C₃ alkyl residue,
R³, R⁴ independently of one another are each H or methyl,
R⁵ is H or a C₁-C₃ alkyl residue,
R⁶ is H, a linear or branched C₁-C₂ alkyl residue or a C₂ alkanoyl residue,
R⁷ is a linear or branched C₁-C₄ alkylene residue or is absent, wherein the chain of the carbon atoms of the alkyl residue can be interrupted by O atoms,
X, Y independently of one another are each O or NR⁸ or are absent,
R⁸ is H or a C₁-C₂ alkyl residue,
PG¹, PG² independently of one other are each H or a (meth)acrylic group, wherein the two residues PG¹ and PG² cannot simultaneously be H;

8. Dental material according to claim 7 in which the variables of Formula I have the following meanings:
R¹, R² are each H,
R³, R⁴ are each H,
R⁵ is H,
R⁶ is H, a linear or branched C₁-C₂ alkyl residue or a C₂ alkanoyl residue,
R⁷ is a linear C₁-C₄ alkylene residue,
X, Y independently of one another are each O or NR⁸ or are absent,
R⁸ is H or a C₁-C₂ alkyl residue,
PG¹, PG² independently of one other are each H or a (meth)acrylic group, wherein the two residues cannot simultaneously be H;

9. Dental material according to one of the preceding claims comprising at least one mono and/or polyfunctional (meth)acrylate, bis-GMA, UDMA, trimethylolpropane trimethacrylate, glycerine dimethacrylate, 1,10-decane diol dimethacrylate, N,N'-diethyl-1,3-bis(acrylamido)propane or a mixture thereof as the additional radically polymerisable monomer.

10. Dental material according to one of the preceding claims which is free of hydrophilic monomers.

11. Dental material according to one of the preceding claims comprising filler with an average particle size of 10 nm to 50 µm.

12. Dental material according to one of the preceding claims comprising as the filler amorphous spherical materials based on oxides or mixed oxides with an average particle size from 10 nm to 1 µm, nanoparticulate or microfine fillers with an average particle size from 10 nm to 500 nm, mini fillers with an average particle size from 0.1 to 5 µm, and/or X-ray-opaque fillers with an average particle size from 10 nm to 500 nm.

13. Dental material according to one of the preceding claims comprising 0.5 wt % water at most.

14. Dental material according to one of the preceding claims comprising boric acid.

15. Use of a dental material according to one of the preceding claims for the preparation of a cement, composite material, filling material, adhesive or coating material.

## Revendications

1. Matériau dentaire polymérisable, qui comprend :
a) au moins un composé polymérisable par voie radicalaire, de formule générale I : 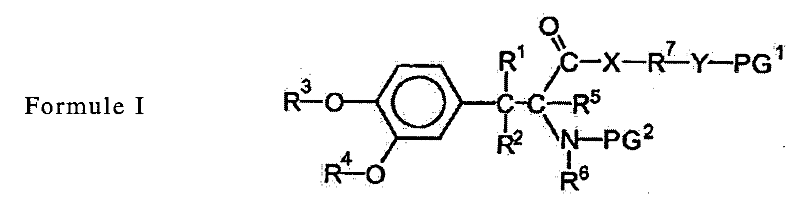 dans laquelle
- R¹ et R² représentent chacun, indépendamment l'un de l'autre, un atome d'hydrogène ou un groupe alkyle en C₁-C₈,
- R³, R⁴ et R⁵ représentent chacun, indépendamment les uns des autres, un atome d'hydrogène ou un groupe alkyle en C₁-C₄,
- R⁶ représente un atome d'hydrogène, un groupe alkyle en C₁-C₁₀ linéaire ou ramifié, ou un groupe alcanoyle en C₁-C₁₀,
- R⁷ ne représente rien ou représente un groupe alcanediyle en C₁-C₁₅ linéaire ou ramifié, étant entendu que la chaîne d'atomes de carbone de ce groupe alkyle peut être interrompue par un ou des atome(s) d'oxygène ou de soufre, mais de préférence, ne représente rien ou représente un groupe alcanediyle en C₁-C₁₅ linéaire ou ramifié,
- X et Y, indépendamment l'un de l'autre, représentent chacun un chaînon symbolisé par O ou NR⁸ ou ne représentent rien,
étant entendu
- que R⁷ ne peut ne rien représenter que si X et/ou Y non plus ne représente(nt) rien,
- que R⁷, X et Y ne peuvent ne rien représenter simultanément que si PG¹ représente un atome d'hydrogène,
- et que R⁸ représente un atome d'hydrogène ou un groupe alkyle en C₁-C₁₀,
- et PG¹ et PG² représentent chacun, indépendamment l'un de l'autre, un atome d'hydrogène ou un groupe apte à une polymérisation par voie radicalaire, étant entendu que les deux entités symbolisées par PG¹ et PG² ne peuvent pas être chacune, simultanément, un atome d'hydrogène ;
b) un amorceur de polymérisation radicalaire ;
c) et une charge.

2. Matériau dentaire conforme à la revendication 1, dans lequel PG¹ et PG² représentent chacun, indépendamment l'un de l'autre, un atome d'hydrogène ou un groupe vinyle, allyle, acroyle ou méthacroyle.

3. Matériau dentaire conforme à la revendication 1 ou 2, qui comprend en outre :
d) un autre monomère polymérisable.

4. Matériau dentaire conforme à l'une des revendications 1 à 3, qui comprend en outre :
e) un solvant.

5. Matériau dentaire conforme à l'une des revendications précédentes, qui comprend :
a) de 0,05 à 40 % en poids d'au moins un composé de formule I ;
b) de 0,01 à 10 % en poids d'un amorceur ;
c) de 1 à 85 % en poids (pour un matériau de revêtement) ou de 20 à 85 % en poids (pour un ciment, un composite) d'une charge ;
d) de 0 à 90 % en poids d'au moins un monomère supplémentaire ;
e) et de 0 à 95 % en poids d'un solvant.

6. Matériau dentaire conforme à la revendication 5, qui comprend :
a) de 1 à 30 % en poids d'au moins un composé de formule I ;
b) de 0,1 à 3,0 % en poids d'un amorceur ;
c) de 1 à 20 % en poids (pour un matériau de revêtement) ou de 20 à 85 % en poids (pour un ciment, un composite) d'une charge ;
d) de 0 à 80 % en poids d'au moins un monomère supplémentaire ;
e) et de 0 à 70 % en poids d'un solvant.

7. Matériau dentaire conforme à l'une des revendications précédentes, dans lequel les symboles de la formule I ont les significations suivantes :
R¹ et R² représentent chacun, indépendamment l'un de l'autre, un atome d'hydrogène ou un groupe alkyle en C₁-C₃,
- R³ et R⁴ représentent chacun, indépendamment l'un de l'autre, un atome d'hydrogène ou un groupe méthyle,
- R⁵ représente un atome d'hydrogène ou un groupe alkyle en C₁-C₃,
- R⁶ représente un atome d'hydrogène, un groupe alkyle en C₁-C₂ linéaire ou ramifié, ou un groupe alcanoyle en C₂,
- R⁷ ne représente rien ou représente un groupe alcanediyle en C₁-C₄ linéaire, étant entendu que la chaîne d'atomes de carbone de ce groupe alkyle peut être interrompue par un ou des atome(s) d'oxygène,
- X et Y, indépendamment l'un de l'autre, représentent chacun un chaînon symbolisé par O ou NR⁸ ou ne représentent rien,
étant entendu que R⁸ représente un atome d'hydrogène ou un groupe alkyle en C₁-C₂,
- et PG¹ et PG² représentent chacun, indépendamment l'un de l'autre, un atome d'hydrogène ou un groupe acroyle ou méthacroyle, étant entendu que ces deux entités ne peuvent pas être chacune, simultanément, un atome d'hydrogène.

8. Matériau dentaire conforme à la revendication 7, dans lequel les symboles de la formule I ont les significations suivantes :
- R¹ et R² représentent chacun un atome d'hydrogène,
- R³ et R⁴ représentent chacun un atome d'hydrogène,
- R⁵ représente un atome d'hydrogène,
- R⁶ représente un atome d'hydrogène, un groupe alkyle en C₁-C₂ linéaire ou ramifié, ou un groupe alcanoyle en C₂,
- R⁷ représente un groupe alcanediyle en C₁-C₄ linéaire,
- X et Y, indépendamment l'un de l'autre, représentent chacun un chaînon symbolisé par O ou NR⁸ ou ne représentent rien,
étant entendu que R⁸ représente un atome d'hydrogène ou un groupe alkyle en C₁-C₂,
- et PG¹ et PG² représentent chacun, indépendamment l'un de l'autre, un atome d'hydrogène ou un groupe acroyle ou méthacroyle, étant entendu que ces deux entités ne peuvent pas être chacune, simultanément, un atome d'hydrogène.

9. Matériau dentaire conforme à l'une des revendications précédentes, qui contient, en tant que monomère supplémentaire polymérisable par voie radicalaire, au moins un acrylate ou méthacrylate monofonctionnel et/ou polyfonctionnel, du bis-GMA, de l'UDMA, du triméthacrylate de triméthylolpropane, du diméthacrylate de glycérol, du diméthacrylate de décane-1,10-diol, du N,N'-diéthyl-1,3-bis(acrylamido)-propane, ou un mélange de ces composés.

10. Matériau dentaire conforme à l'une des revendications précédentes, qui ne contient pas de monomères hydrophiles.

11. Matériau dentaire conforme à l'une des revendications précédentes, qui contient une charge présentant une taille moyenne de particules de 10 nm à 50 µm.

12. Matériau dentaire conforme à l'une des revendications précédentes, qui contient, en tant que charge, des matériaux amorphes à base d'oxydes ou d'oxydes mixtes, en particules de forme sphérique et présentant une taille moyenne de particules de 10 nm à 1 µm, des charges nanoparticulaires ou microfines présentant une taille moyenne de particules de 10 nm à 500 nm, des minicharges présentant une taille moyenne de particules de 0,1 à 5 µm, ou des charges opaques aux rayons X et présentant une taille moyenne de particules de 10 nm à 500 nm.

13. Matériau dentaire conforme à l'une des revendications précédentes, qui contient au maximum 0,5 % en poids d'eau.

14. Matériau dentaire conforme à l'une des revendications précédentes, qui contient de l'acide borique.

15. Utilisation d'un matériau dentaire conforme à l'une des revendications précédentes pour la fabrication d'un ciment, d'un matériau composite, d'un matériau de remplissage, d'un matériau adhésif ou d'un matériau de revêtement.
